(19) Europäisches Patentamt
European Patent Office
Office européen des brevets

(11) **EP 0 983 385 B1**

(12) **EUROPEAN PATENT SPECIFICATION**

(45) Date of publication and mention of the grant of the patent:
**31.03.2010 Bulletin 2010/13**

(21) Application number: **98922938.0**

(22) Date of filing: **21.05.1998**

(51) Int Cl.:
*C12Q 1/68* (2006.01)

(86) International application number:
**PCT/GB1998/001481**

(87) International publication number:
**WO 1998/054359 (03.12.1998 Gazette 1998/48)**

(54) **PREDICTION OF INFLAMMATORY DISEASE ASSOCIATED WITH IL-1 GENELOCI POLYMORPHISMS**

VORHERSAGE VON ENTZÜNDLICHEN KRANKEITEN ASSOZIERT MIT IL-1 GENLOCUS POLYMORPHISMEN

PREDICTION D'UNE MALADIE INFLAMMATOIRE ASSOCIEE AUX POLYMORPHISMES DES LOCI SUR LE GENE DE L'IL-1

(84) Designated Contracting States:
**AT BE CH CY DE DK ES FI FR GB GR IE IT LI LU MC NL PT SE**

(30) Priority: **29.05.1997 GB 9711040**

(43) Date of publication of application:
**08.03.2000 Bulletin 2000/10**

(73) Proprietor: **Interleukin Genetics, Inc.**
**Waltham, MA 02452 (US)**

(72) Inventors:
• **Duff, Gordon**
  **Sheffield S10 3BZ (GB)**
• **Cox, Angela**
  **Crookes, Sheffield S10 1NS (GB)**
• **Camp, Nicola, Jane**
  **Hillsbourough, Sheffield (GB)**
• **De Giovine, Francesco, Saverio**
  **Ranmoor, Sheffield S10 3GZ (GB)**

(74) Representative: **Crump, Julian Richard John et al**
**Mintz, Levin, Cohn, Ferris, Glovsky and Popeo IP, LLP**
**Alder Castle**
**10 Noble Street**
**London EC2V 7JX (GB)**

(56) References cited:
**WO-A-97/38135     WO-A-97/43446**
**WO-A-98/15653**

• MCDOWELL T.L. ET AL.,: "A gentic association between juvenile rheumatoid arthritis and a novel interleukin-1 alpha polymorphism" ARTHRITIS & RHEUMATISM, vol. 38, no. 2, - February 1995 pages 221-228, XP002077314 cited in the application
• DI GIOVINE F.S. ET AL.,: "Single base polymorphism at -511 in the human interleukin-1beta gene (IL1beta)" HUMAN MOLECULAR GENETICS, vol. 1, no. 6, - September 1992 page 450 XP002077315 cited in the application
• CLAY F. E. ET AL.,: "Novel interleukin-1 receptor antagonist exon polymorphisms and their use in allele-specific mRNA assessment" HUM. GENET., vol. 97, - June 1996 pages 723-726, XP002077312 cited in the application
• EPPLEN C. ET AL.,: "Dinucleotide repeat polymorphism in the human IL1A gene" HUMAN MOLECULAR GENETICS, vol. 3, no. 9, - September 1994 page 1710 XP002077310
• GUASCH J. F. ET AL.,: "Five novel intragenic dimorphisms in the human interleukin-1 genes combine to high informativity" CYTOKINE, vol. 8, no. 8, - August 1996 pages 598-602, XP002077311
• BAILLY S. ET AL.,: "Genetic polymorphism of human interleukin-1alpha" EUR. J. IMMUNOL., vol. 23, - June 1993 pages 1240-1245, XP002077313
• TODD S. & NAYLOR S.L.: "Dinucleotide repeat polymorphism in the human interleukin 1, alpha gene (IL1A)" NUCLEICS ACIDS RESEARCH, vol. 19, no. 13, - 1991 page 373756 XP002077341 cited in the application

EP 0 983 385 B1

- COX A. ET AL.,: "AN analysis of linkage disequilibrium in the interleukin-1 gene cluster, using a novel grouping method for multiallelic markers" AM. J. HUM. GENET. , vol. 62, - May 1998 pages 1180-1188, XP002077316

**Description**

**Background**

1. Field of the Invention

This invention relates to methods and kits for the identification of susceptibility to immunoinflammatory disorders or disorders modulated by IL-1 biologic activity. Specifically, the method involves the detection of at least one allele in an IL-1 haplotype, such as the IL-1 (44112332) haplotype, which is associated with inflammatory disorders, such as coronary artery disease, osteoporosis, nephropathy in diabetes mellitus, alopecia areata, Graves disease, systemic lupus erythamatosus, lichen sclerosis, ulcerative colitis, diabetic retinopathy, periodontal disease, juvenile chronic arthritis, particularly chronic iridocyclitis, psoriasis, insulin dependent diabetes in DR3/4 patients, asthma, chronic inflammatory liver diseases, chronic inflammatory lung disease, lung fibrosis, liver fibrosis, as well as acute and chronic inflammatory diseases of the central nervous system, the cardiovascular system, the gastrointestinal system, the musculoskeletal system, the endocrine system, the skin and associated structure, the immune system, the hepato-biliary system, or any site in the body where pathology can occur with an "immune-inflammatory" component involving IL gene products irrespective of the initiating facctors, eg: infectious agents, trauma, neoplasia, autoimmunity, idiopathic diesases, biologic toxins and noxious agents, and other diseases with an IL-1 loci genetic component.

2. Description of the Background

Genetic testing (also called genetic screening, genotyping or molecular diagnostics) can be defined broadly as the testing of nucleic acid of a patient in an analytical capacity to determine if a patient contains mutations (or alleles or polymorphisms) that either cause or increase susceptibility to a disease state or are in "linkage disequilibrium" with the gene causing a disease state.

Linkage disequilibrium, refers to the tendency of specific alleles to occur together more frequently than would be expected by chance. Alleles at given loci are in equilibrium if the frequency of any particular set of alleles (or haplotype) is the product of their individual population frequencies. The cause of disequilibrium is often unclear. It can be due to selection for certain allele combinations, or to recent admixture of genetically heterogeneous populations. In addition, in the case of markers that are very tightly linked to a disease gene, an association of an allele (or group of linked alleles) with the disease gene is expected if the disease mutation occurred in the recent past, so that sufficient time has not elapsed for equilibrium to be achieved through recombination events in that small chromosomal region.

The early detection of a predisposition to genetic diseases presents the best opportunity for medical intervention. Early genetic diagnosis may improve the prognosis for a patient through supervision and early intervention before the clinically detectable disorder occurs. In cases where patients with similar symptoms are treated with variable success, sophisticated genetic testing can differentiate individual patients with subtle or undetectable differences and can lead to more suitable individual treatments. Early intervention may involve methods such as gene therapy or treatment with IL-1 modulators. With the development of genetic testing, it is now possible to identify gene mutations which indicate a propensity to develop disease, even when the disease is of polygenic origin. The number of diseases that can be diagnosed by molecular biological methods continues to grow with increased understanding of the genetic basis of multifactorial disorders (*see e.g.*, United States Patent Nos. 4,582,788; 5,110,920; 4,801,531; 4,666,828; and 5,268,267).

**The Il-1 Gene Cluster**

The IL-1 gene cluster is located on the long arm of human chromosome 2 (2q13) and contains at least the genes for IL-1α (IL1A), IL-1β (IL1B), and the IL-1 receptor antagonist (IL1RN) within a region of 430 Kb (Nicklin, et al., Genomics 19: 382-4 (1994)). The agonist molecules, IL-1α and IL-1β, have potent pro-inflammatory activity and are at the head of many inflammatory cascades. Their actions, often via the induction of other cytokines such as IL-6 and IL-8, lead to activation and recruitment of leukocytes into damaged tissue, local production of vasoactive agents, fever response in the brain and the hepatic acute phase response. All three IL-1 molecules bind to type I and to type II IL-1 receptors, but only the type I receptor transduces a signal to the interior of the cell. In contrast, the type II receptor is shed from the cell membrane and acts as a decoy receptor. The receptor antagonist and the type II receptor, therefore, are both antiinflammatory in their actions.

Inappropriate production of IL-1 appears to play a central role in the pathology of many autoimmune and inflammatory diseases, including rheumatoid arthritis, inflammatory bowel disorder, psoriasis, and others. In addition, there are stable inter-individual differences in the rates of production of IL-1, and some of this variation may be accounted for by genetic differences at IL-1 gene loci (Molvig, et al., Scand. J. Immunol. 27:705-16 (1988); Pociot, et al., Eur. J. Clin. Invest. 22: 396-402 (1992)).Thus, the IL-1 genes are reasonable candidates for determining part of the genetic susceptibility to inflammatory diseases, most of which have a multifactorial etiology with a polygenic component.

Certain alleles from the IL-1 gene cluster are known to be associated with particular disease states. For example, we have shown that IL1RN allele 2 is associated with coronary artery disease, osteoporosis (U.S. Application Nos. 08/813,416, 08/628,282), nephropathy in diabetes mellitus (Blakemore, et al., Hum. Genet. 97(3): 369-74 (1996)), alopecia areata (Cork, et al., J. Invest. Dermatol. 104(5 Supp.): 15S-16S (1995)), Graves disease (Blakemore, et al., J. Clin. Endocrinol. 80(1): 111-5 (1995)), systemic lupus erythamatosus (Blakemore, et al., Arthritis Rheum. 37: 1380-85 (1994)), lichen sclerosis (Clay, et al., Hum. Genet. 94: 407-10 (1994)), and ulcerative colitis (Mansfield, et al., Gastoenterol. 106(3):637-42 (1994). IL1RN cluster allele 1 is associated with diabetic retinopathy (WO/98/15653). Likewise, we have shown that the IL1A allele 2 from marker -889 and IL1B(TaqI) allele 2 from marker +3953 are associated with peridontal disease (U.S. Patent No. 5,686,246). The allele 2 from marker -889 is also associated with juvenile chronic arthritis, particularly chronic iridocyclitis (McDowell, el al., Arthritis Rheum. 38: 221-28 (1995)). The IL1B(TaqI) Allele 2 from marker +3953 of IL1B is also associated with psoriasis and insulin dependent diabetes in DR3/4 patients (di Giovine, et al., Cytokine 7: 606 (1995); Pociot, et al., Eur J. Clin. Invest. 22: 396-402 (1992)).

Summary of the Invention

The present invention provides a novel method for the early prediction of a propensity to develop inflammatory disorders. It also provides kits for the early determination of said propensity.

The method of predicting increased risk for disease modulated by IL-1 biologic activity (herein termed inflammatory disorders) consists of detecting the presence of one or more alleles of the IL-1 (44112332) haplotype, or alternatively, the entire haplotype may be detected. Having one or more of the alleles in an IL-1 haplotype indicates increased risk for a variety of inflammatory disorders. These include, but are not limited to, coronary, artery disease, osteoporosis, nephropathy in diabetes mellitus, alopecia areata, Graves disease, systemic lupus erythamatosus, lichen sclerosis, ulcerative colitis, periodontal disease, juvenile chronic arthritis particularly chronic iridocyclitis, psoriasis, insulin dependent diabetes in DR3/4 patients, diabetic retinopathy and other diseases with an IL-1 loci genetic component.

The IL-1 haplotype is associated with the following diseases: coronary artery disease, osteoporosis, nephropathy in diabetes mellitus, alopecia areata, Graves disease, systemic lupus erythamatosus, lichen sclerosis, and ulcerative colitis. The IL-1 (33221461) haplotype is associated with the following diseases: periodontal disease, juvenile chronic arthritis, psoriasis, insulin dependent diabetes, and diabetic retinopathy.

In another embodiment, the invention can be described as the following: isolating nucleic acid from the patient, identifying one or more alleles present in the IL-1 gene cluster, and comparing the one or more alleles to a control sample. The control sample contains at least one allele from an IL-1 haplotype. Thus, a control sample contains one or more of the following alleles from the IL-1 (44112332) haplotype:

| | |
|---|---|
| allele 4 of the 222/223 marker | (* 132 mu PCR product), |
| allele 4 of the gz5/gz6 marker | (*91 mu PCR product), |
| allele 1 of the -889 marker | (contains an NcoI site), |
| allele 1 of the +3953 marker | (contains two TaqI sites), |
| allele 2 of the -511 marker | (contains a Bsu36I site), |
| allele 3 of the gaat.p33330 marker | (*197 mu PCR product), |
| allele 3 of the Y31 marker | (*160 mu PCR product), |
| allele 2 of the VNTR marker | (240 bp PCR product). |

> * The sizes of microsatellite marker PCR products is given in mobility units which approximate the size in base pairs, but may vary according to the method of determination, as would be readily ascertained by one of skill in the art. The markers positions are as indicated by in the references of Table 1.

Similarity of the identified alleles from the patient to the control sample indicates the patient's predisposition to inflammatory disease. The control sample may also be an allelic ladder comprised of a plurality of the above listed alleles and may also contain additional alleles from the above markers. There may be a plurality of control samples, each containing different alleles or sets of alleles.

Another embodiment of the invention is a kit for the detection of alleles that are predictive of inflammatory disease. The kit generally includes at least one oligonucleotide complementary to a DNA sequence in the IL-1 gene family and a control sample. The control sample contains an allele known to be associated with inflammatory disease, as described above. The control sample may contain the actual PCR products produced by amplification of said alleles, or alternatively may contain genomic or cloned DNA from an individual that carries an IL-1 haplotype.

The kit may also include a DNA sampling means, a DNA purification means, and PCR reagents. Further, the oligonucleotide may contain a detectable label. The kit comprises at least two oligonucleotides selected from the group consisting of:

ATGTATAGAATTCCATTCCTG (SEQ ID NO. 1),
TAAAATCAAGTGTTGATGTAG (SEQ ID NO. 2),
GGGATTACAGGCGTGAGCCACCGCG (SEQ ID NO. 3),
TTAGTATTGCTGGTAGTATTCATAT (SEQ ID NO. 4),
TGTTCTACCACCTGAACTAGG (SEQ ID NO. 5),
TTACATATGAGCCTTCCATG (SEQ ID NO. 6),
CTCAGGTGTCCTCGAAGAAATCAAA (SEQ ID NO. 7),
GCTTTTTTGCTGTGAGTCCCG (SEQ ID NO. 8),
TGGCATTGATCTGGTTCATC (SEQ ID NO. 9),
GTTTAGGAATCTTCCCACTT (SEQ ID NO. 10),
GAGGCGTGAGAATCTCAAGA (SEQ ID NO. 11),
GTGTCCTCAAGTGGATCTGG (SEQ ID NO. 12),
GGGCAACAGAGCAATGTTTCT (SEQ ID NO. 13),
CAGTGTGTCAGTGTACTGTT (SEQ ID NO. 14),
CTCAGCAACACTCCTAT (SEQ ID NO. 15),
TCCTGGTCTGCAGGTAA (SEQ ID NO. 16), and
TTACGCAGATAAGAACCAGTTTGG (SEQ ID NO. 17).

Other embodiments and advantages of the invention are set forth in part in the description which follows, and will be obvious from this description. More generally, the description below also describes aspects and embodiments of the disclosure of the present application.

## Description of the drawings

Figure 1 depicts the 8 markers in the IL-1 gene cluster and their approximate location.
Figure 2 depicts the correlation between linkage disequilibrium and physical distance.

## Detailed description of the invention and of the disclosure

As embodied and broadly described herein, the present invention and the present disclosure is directed to methods for the detection of at least one allele of an IL-1 haplotype, including the IL-1 (44112332) haplotype or the IL-1 (33221461) haplotype, that is associated with inflammatory disorders.

The term 'marker,' as used herein, refers to a specific site in the genome which exhibits sequence variations between individuals. For example, herein are described at least 8 markers: the 222/223, gz5/gz6, -889, +3953, -511, gaat.p33330, Y31 and VNTR markers.

The term 'allele' refers to the different sequence variants found at given markers. For example, there are at least 5 alleles, 1 through 5, at the VNTR marker and there are at least 2 alleles at the -889 marker. The sequence variants may be single or multiple base changes, including insertions, deletions or substitutions or may be variable number of sequence repeats and the like.

The term 'linkage disequilibrium' refers to the co-inheritance of two alleles at frequencies greater than would be expected from the separate frequencies of occurrence of each allele in a given control population. The expected frequency of occurrence of two alleles that are inherited independently is the frequency of the first allele multiplied by the frequency of the second allele. Alleles that co-occur at expected frequencies are said to be in 'linkage equilibrium.'

The term 'haplotype' is a set of alleles that are inherited together as a group (are in linkage disequilibrium). As used herein, haplotype is defined to include those haplotypes that occur at statistically significant levels ($p_{corr} \leq 0.05$).

As used herein the phrase 'an IL-1 haplotype' refers to any haplotype in the IL-1 loci. It includes at least the IL-1 (44112332) haplotype and the (33221461) haplotype as described herein.

As used herein, the phrase 'IL-1 (44112332) haplotype' refers to the haplotype consisting of at least:

allele 4 of the 222/223 marker of IL1A;
allele 4 of the gz5/gz6 marker of IL1A;
allele 1 of the -889 marker of IL1A;
allele 1 of the +3953 marker of IL1B;

allele 2 of the -511 marker of IL1B;
allele 3 of the gaat.p33330 marker;
allele 3 of the Y31 marker; and
allele 2 of the VNTR marker of IL1RN.

The phrase 'IL-1 (44112332) haplotype' also includes any additional alleles that are in linkage disequilibrium with this set of alleles. For example, the IL-1 (44112332) haplotype may also contain the following alleles:

| | |
|---|---|
| allele 2 of the 1731 marker of the IL1RN gene | (A at position 1731), |
| allele 2 of the 1812 marker of the IL1RN gene | (A at position 1812), |
| allele2 of the 1868 marker of the IL1RN gene | (G at position 1868), |
| allele 2 of the 1887 marker of the IL1RN gene | (C at position 1887), |
| allele 2 of the 8006 marker of the IL1RN gene | (contains an HpaII or MspI site), |
| allele 2 of the 8061 marker of the IL1RN gene | (lacks an MwoI site), and |
| allele 2 of the 9589 marker of the IL1RN gene | (contains an SspI site). |

As used herein, the phrase 'IL-1 (33221461) haplotype' refers to the haplotype consisting of at least:

allele 3 of the 222/223 marker of IL1A;
allele 3 of the gz5/gz6 marker of IL1A;
allele 2 of the -889 marker of IL1A;
allele 2 of the +3953 marker of IL1B;
allele 1 of the -511 marker of IL1B;
allele 4 of the gaat.p33330 marker;
allele 6 of the Y31 marker; and
allele 1 of the VNTR marker of IL1RN.

The phrase 'IL-1 (33221461) haplotype' also includes any additional alleles that are in linkage disequilibrium with this set of alleles.

The phrase 'inflammatory disease' or 'inflammatory disorder,'as used herein, refers to those diseases associated with inheritance of an IL-1 haplotype. In addition to conventional diseases of inflammation, the term encompasses those diseases which are not traditionally thought of as inflammatory conditions, but which have an inflammatory component or are modulated by the IL-1 gene cluster, including certain infectious diseases, certain metabolic disorders, and certain aspects of tumor growth, spread and control. Inflammatory disorders include, but are not limited to, coronary artery disease, osteoporosis, nephropathy in diabetes mellitus, alopecia areata, Graves disease, systemic lupus erythamatosus, lichen sclerosis, ulcerative colitis, diabetic retinopathy, periodontal disease, juvenile chronic arthritis particularly chronic iridocyclitis, psoriasis, insulin dependent diabetes in DR3/4 patients and other diseases with an IL-1 loci genetic component.

As used herein, the process of 'detecting alleles' is variously described as 'genotyping, determining or identifying an allele or polymorphism,' or any similar phrase. The allele actually detected might be a disease-causing mutation, or a mutation that is in linkage disequilibrium with a disease-causing mutation. It will be manifest in the genomic DNA of a patient, but may also be detectable from RNA or protein sequences transcribed or translated from this region.

The phrase 'IL-1 gene cluster' or 'IL-1 loci' or 'IL-1 gene family,' as used herein, includes all the nucleic acid at or near 2q13 on chromosome 2, including the ILIA, IL1B and IL1RN genes and any other linked sequences.

By 'propensity,' 'predisposition' or 'susceptibility' for disease what is meant is that certain alleles are hereby discovered to be 'associated' with a given disease state. They are thus over represented in individuals with disease as compared to healthy individuals and indicate that these individuals are at higher risk for developing disease or may develop a more severe form or particular subset of disease type.

By 'microsatellite marker' what is meant is a marker whose alleles comprise different numbers of short repeat (< 5 bp) sequences.

By 'VNTR marker' what is meant is a marker whose alleles comprise different numbers of medium length repeat sequences. A specific VNTR marker from intron 2 of the IL1RN gene is described herein.

By 'biallelic marker' what is meant is a marker with only two known alleles.

The cytokines in the IL-1 gene cluster play a central role in the immune and inflammatory responses as well as a number of inflammatory diseases. The degree of linkage disequilibrium of the IL-1 gene cluster was investigated and found to be statistically significant at 95% confidence level across an approximately 400 Kb stretch of the region.

A previously unrecognized IL-1 (44112332) haplotype and a IL-1 (33221461) haplotype were found in the healthy population. The entire IL-1 (44112332) haplotype was found to be at an increased frequency in patients with nephropathy in diabetes mellitus.

The disclosure is directed to a method of predicting the propensity or predisposition of a patient to inflammatory disease by genotyping the patient's DNA at the IL-1 gene cluster. The patient's genotype is compared with a control sample that contains one or more alleles from an IL-1 haplotype, such as the IL-1 (44112332) haplotype or the IL-1 (33221461) haplotype. The alleles include:

| | |
|---|---|
| allele 4 of the 222/223 marker | (* 132 mu PCR product), |
| allele 4 of the gz5/gz6 marker | (*91 mu PCR product), |
| allele 1 of the -889 marker | (contains an NcoI site), |
| allele 1 of the +3953 marker | (contains two TaqI sites), |
| allele 2 of the -511 marker | (contains a Bsu36I site), |
| allele 3 of the gaat.p33330 marker | (*197 mu PCR product), |
| allele 3 of the Y31 marker | (*160 mu PCR product), |
| allele 2 of the VNTR marker | (240 bp PCR product). |

*The sizes of microsatellite marker PCR products is given in mobility units which approximate the size in base pairs, but may vary according to the method of determination, as would be readily ascertained by one of skill in the art.

The control sample may also contain the following alleles which may be part of the IL-1 (44112332) haplotype:

| | |
|---|---|
| allele 2 of the 1731 marker of the IL1RN gene | (A at position 1731), |
| allele 2 of the 1812 marker of the IL1RN gene | (A at position 1812), |
| allele 2 of the 1868 marker of the IL1RN gene | (G at position 1868), |
| allele 2 of the 1887 marker of the IL1RN gene | (C at position 1887), |
| allele 2 of the 8006 marker of the IL1RN gene | (contains an HpaII or MspI site), |
| allele 2 of the 8061 marker of the IL1RN gene | (lacks an MwoI site), |
| allele 2 of the 9589 marker of the IL1RN gene | (contains an SspI site). |

The control sample may also contain the following alleles from the IL-1 (33221461) haplotype:

| | |
|---|---|
| allele 3 of the 222/223 marker | (*130 mu PCR product), |
| allele 3 of the gz5/gz6 marker | (*88 mu PCR product), |
| allele 2 of the -889 marker | (lacks an NcoI site), |
| allele 2 of the +3953 marker | (contains a TaqI site), |
| allele 1 of the -511 marker | (lacks a Bsu36I site), |
| allele 4 of the gaat.p33330 marker | (*201 mu PCR product), |
| allele 6 of the Y31 marker | (*166 mu PCR product), |
| allele 1 of the VNTR marker | (412 bp PCR product). |

These alleles are known to be associated with a variety of inflammatory disorders, such as coronary artery disease, osteoporosis, nephropathy in diabetes mellitus, alopecia areata, Graves disease, systemic lupus erythamatosus, lichen sclerosis, ulcerative colitis, diabetic retinopathy, periodontal disease, juvenile chronic arthritis particularly chronic iridocyclitis, psoriasis, insulin dependent diabetes in DR3/4 patients and other diseases with an IL-1 loci genetic component.

The identification of a disease-associated haplotype (the set of alleles in linkage disequilibrium) will allow the investigator to begin to determine which of the alleles are causative, rather than merely linked to a disease-causing allele. This information can be used to design rational treatment approaches, including gene therapy and treatment with a variety of agents that modulate the activity of the proteins produced by the IL-1 gene cluster. It is also possible that multiple mutations might each partially contribute to disease states for diseases of complex origin. Thus, if the mutations are additive or synergistic, the detection of these mutations would indicate even higher risk for the disease. Furthermore, the presence of disease-associated alleles in linkage disequilibrium will allow the diagnostic testing of more than one allele, which will help to eliminate the possibility of false positive results.

Techniques for determining the presence of particular alleles may be nucleic acid techniques based on size or sequence, such as restriction fragment length polymorphism (RFLP), nucleic acid sequencing, or hybridization.

These techniques may also comprise the step of amplifying the nucleic acid before analysis. Amplification techniques are known to those of skill in the art and include cloning, polymerase chain reaction (PCR), polymerase chain reaction of specific alleles (PASA), polymerase chain ligation nested polymerase chain reaction, and the like. Amplification products may be assayed in a variety of ways, including size analysis, restriction digestion followed by size analysis, detecting specific tagged oligonucleotide primers in the reaction products, allele-specific oligonucleotide (ASO) hybridization, sequencing, hybridization, and the like.

PCR based detection means include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be detected. One means of analyzing multiple markers involves labeling each marker with a different fluorescent probe. The PCR products are then analyzed on a fluorescence based automated sequencer. Of course, hybridization based detection allows the differential detection of multiple PCR products in a sample. Other techniques are known in the art to allow multiplex analyses of a plurality of markers.

Allele detection techniques may be protein based if a particular allele produces a protein with an amino acid variant. For example, epitopes specific for the amino acid variant can be detected with monoclonal antibodies. Likewise, it is possible to detect alleles if they are present in processed RNA by techniques that are known in the art.

Another embodiment of the invention is directed to diagnostic kits for detecting a propensity for inflammatory disease in a patient. The kits can be used pre- or post-symptomatically or prenatally.

A number of assay formats are useful for genotyping using the provided oligonucleotides. The most common formats involve nucleic acid binding, such binding to filters, beads, or microtiter plates and the like. Techniques may include dot blots, RNA blots, DNA blots, PCR, RFLP, and the like.

The oligonucleotides may be a variety of natural and synthetic compositions such as synthetic oligonucleotides, restriction fragments, cDNAs, synthetic PNAs, and the like. The assay may also employ labeled oligonucleotides to allow ease of identification in the assays. Examples of labels which may be employed include radiolabels, enzymes, fluorescent compounds, streptavidin, avidin, biotin, magnetic moieties, metal binding moieties, antigen or antibody moieties, and the like. The kits of the invention comprise at least two oligonucleotides selected from the group consisting of:
ATGTATAGAATTCCATTCCTG (SEQ ID NO. 1),
TAAAATCAAGTGTTGATGTAG (SEQ ID NO. 2),
GGGATTACAGGCGTGAGCCACCGCG (SEQ ID NO. 3),
TTAGTATTGCTGGTAGTATTCATAT (SEQ ID NO. 4),
TGTTCTACCACCTGAACTAGG (SEQ ID NO. 5),
TTACATATGAGCCTTCCATG (SEQ ID NO. 6),
CTCAGGTGTCCTCGAAGAAATCAAA (SEQ ID NO. 7),
GCTTTTTTGCTGTGAGTCCCG (SEQ ID NO. 8),
TGGCATTGATCTGGTTCATC (SEQ ID NO. 9),
GTTTAGGAATCTTCCCACTT (SEQ ID NO. 10),
GAGGCGTGAGAATCTCAAGA (SEQ ID NO. 11),
GTGTCCTCAAGTGGATCTGG (SEQ ID NO. 12),
GGGCAACAGAGCAATGTTTCT (SEQ ID NO. 13),
CAGTGTGTCAGTGTACTGTT (SEQ ID NO. 14),
CTCAGCAACACTCCTAT (SEQ ID NO. 15),
TCCTGGTCTGCAGGTAA (SEQ ID NO. 16), and
TTACGCAGATAAGAACCAGTTTGG (SEQ ID NO. 17).

The kit may also include DNA sampling means such as the AmpliCard™ (University of Sheffield, Sheffield, England S 10 2JF; Tarlow JW, et al. J. of Invest. Dermatol. 103:387-389 (1994)) and the like; DNA purification means such as cell lysis with SDS followed by proteinase K digestion, Nucleon™ kits, and the like; and PCR reagents, such as 10X reaction buffers, thermostable polymerase, dNTPs, and the like.

Another embodiment of the disclosure provides a method of identifying additional alleles in linkage with an IL-1 haplotype, for example the IL-1 (44112332) haplotype. Additional alleles from the IL-1 gene cluster can be identified by sequence analysis of this region of the DNA. Alternatively, additional alleles can be identified by restriction enzyme analysis (for methods of identifying novel markers, see McDowell, et al., Br. J. Rheumatol. 32(Supp 1): 182 (1983); Wilson, et al., Hum. Molec. Genet. 1(5): 353 (1992); di Giovine, et al., Hum. Molec. Genet. 1(6): 450 (1992); and our paper, Clay, et al., Human Genetics 97(6); 723-26 (1996)). Sequencing or restriction enzyme analysis of the IL-1 gene cluster DNA from an IL-1 (44112332) haplotype carrier and comparison with sequences from non-carrier individuals will identify additional alleles that may be present in the haplotype. Alternatively, linkage disequilibrium can be established as described in Example 3, or by family studies.

The disclosure is also directed to methods for identifying additional inflammatory disorders that are associated with alleles from an IL-1 haplotype. Groups of patients with and without disease are genotyped and compared. In this way it is possible to show that the haplotype is associated with new inflammatory disorders.

The following example illustrates embodiments of the invention and of the disclosure but should not be viewed as limiting the scope of the invention.

## Example 1 Genotyping

All human subjects were unrelated, Caucasian, healthy blood donors from Sheffield (n = 112). Subjects were typed at the loci indicated in Table 1.

**Table 1. Markers Used in Haplotype Study**

| Marker | Gene | Reference |
|---|---|---|
| 222/223 | IL1A | Todd & Naylor, Nucleic Acids Res. 19: 3756 (1991) |
| gz5/gz6 | IL1A | Zuliani, et al., Am. J. Hum. Genet. 46: 963-69 (1990) |
| -889 | IL1A | McDowell, et al., Arth. & Rheum. 38:221-8 (1995) |
| +3953 | IL1B | di Giovine, et al., Cytokine 7(6): 606 (1995) |
| -511 | IL1B | di Giovine, Hum. Molec. Genet. 1(6):450 (1992) |
| gaat.p33330 | between IL1B and IL1RN | Murray, *et al*., Coop. Hum. Link. Center, unpublished |
| Y31 | between IL1B and IL1RN | Spurr, et al., Cytogenet. & Cell Genet. 73: 255-73 (1996) |
| VNTR | IL1RN | Tarlow, et al., Hum. Genet. 91: 403-4 (1993) |

The primer sequences and fluorescent labels used in PCR amplification of markers were as in Table 2.

**Table 2. Primer Sequence and Florescent Label for Genotyping**

| Marker | Label | Primer Sequence | |
|---|---|---|---|
| 222/223 | HEX | ATGTATAGAATTCCATTCCTG | (SEQ ID NO. 1) |
| | | TAAAATCAAGTGTTGATGTAG | (SEQ ID NO. 2) |
| gz5/gz6 | FAM | GGGATTACAGGCGTGAGCCACCGCG | (SEQ ID NO. 3) |
| | | TTAGTATTGCTGGTAGTATTCATAT | (SEQ ID NO. 4) |
| -889 | NONE | TGTTCTACCACCTGAACTAGG | (SEQ ID NO. 5) |
| | | TTACATATGAGCCTTCCATG | (SEQ ID NO. 6) |
| +3953 | NONE | CTCAGGTGTCCTCGAAGAAATCAAA | (SEQ ID NO. 7) |
| | | GCTTTTTTGCTGTGAGTCCCG | (SEQ ID NO. 8) |
| -511 | NONE | TGGCATTGATCTGGTTCATC | (SEQ ID NO. 9) |
| | | GTTTAGGAATCTTCCCACTT | (SEQ ID NO. 10) |
| gaat.p33330 | FAM | GAGGCGTGAGAATCTCAAGA | (SEQ ID NO. 11) |
| | | GTGTCCTCAAGTGGATCTGG | (SEQ ID NO. 12) |
| Y31 | HEX | GGGCAACAGAGCAATGTTTCT | (SEQ ID NO. 13) |
| | | CAGTGTGTCAGTGTACTGTT | (SEQ ID NO. 14) |
| VNTR | NONE | CTCAGCAACACTCCTAT | (SEQ ID NO. 15) |
| | | TCCTGGTCTGCAGGTAA | (SEQ ID NO. 16) |

Reaction conditions were as described in Table 3.

**Table 3. Reaction Conditions**

| Marker | Conditions |
|---|---|
| 222/223 | 50 mM KCl, 10 mM Tris-HCl pH 9.0,1.5 mM MgCl$_2$, 200 :mM dNTPs. 25 ng primers, 50 ng template, 0.004% W-1 (Gibco-BRL) 0.2 u Taq, PCR was done at 30 cycles of 94˚C for 1", 55˚C for 1", 72˚C for 1" |
| gz5/gz6 | as per marker 222/223, except 1 u of Perfect Match (StrataGene) was added |
| -889 | per marker 222/223, except PCR was done for 1 cycles at 96˚C for 1", 40 cycles of 94˚C for 1", 46˚C for 1" 72˚C for 1" and 1 cycle of 72˚C for 4", products were cleaved with NcoI for analysis |
| +3953 | as per marker 222/223, except PCR was done for 35 cycles with annealing at 67.5˚C, products were cleaved with Taq 1 for analysis |
| -511 | as per marker 222/223, except PCR was done for 1 cycle at 95˚C for 2", 35 cycles of 95˚C for 1", 53˚C for 1" 74˚C for 1" and 1 cycle of 74˚C for 4", products were cleaved with AvaI and Bsu36I for analysis |
| gaat.p33330 | per marker 222/223 |
| Y31 | per marker 222/223 |
| VNTR | per marker 222/223 except with 1.7 mM MgCl$_2$, 1 cycle at 96˚C for 1"; 30 cycles of 94˚C for 1", 60˚C for 1", 70˚C for 1" and 1 cycles at 70˚C for 2" |

222/223, gz5/gz6, gaat.p33330 and Y31 PCR products were examined by agarose gel electrophoresis and the remainder of the PCR products were pooled according to the intensity of ethidium bromide staining. 2 $\mu$l of the pool was analyzed on an ABI 373A automated sequencer and allele sizes were determined using the Genescan and Genotyper software. Alleles were globally binned using a simple computer program and numbered in order of size.

-889 PCR products were digested with NcoI and the resulting fragments sized on 8% PAGE. Allele 1 produces 83 and 16 bp fragments. Allele 2 produces a 99 bp fragment.

+3953 PCR products were digested with restriction enzyme Taq I. Allele 1 produces fragments of 97, 85 and 12 bp, and allele 2 produces fragments of 182 and 12 bp.

-511 PCR products were digested with AvaI and Bsu36I and the fragments were sized by 8% PAGE. Allele 1 produces 190 and 114 bp fragments when digested with AvaI and a 304 bp fragment when digested with Bsu36I. Allele 2 produces a 304 bp fragment when digested with AvaI and 190 and 114 bp fragments when digested with Bsu36I.

VNTR PCR products were sized by electrophoresis on 2% agarose gel at 90V for 45 minutes. Allele 1 has 4 repeats and the PCR product is 412 bp, allele 2 has 2 repeats and the PCR product is 240 bp, allele 3 has 3 repeats and the PCR product is 326 bp, allele 4 has 4 repeats and the PCR product is 498 bp, allele 5 has 6 repeats and the PCR product is 584 bp.

Intergenic distances were determined by estimation based on the insert sizes of relevant PAC clones from a contig spanning the IL-1 gene cluster (Nicklin, et al., Genomics 19: 382-4 (1994); Nothwang et al. Genomics (in press)). Intragenic distances were determined from the relevant nucleotide sequence obtained form the GENBANK database.

**Example 2 <u>Method for Estimating Linkage Disequilibrium</u>**

Because four of the markers studied herein are multiallelic, a preliminary analysis was carried out to determine which allelic combinations between pairs of loci contributed to the greatest disequilibrium, in order that the disequilibrium would not be masked when the alleles were grouped into biallelic systems. The E.H. program of Xie and Ott (Handbook of Human Genetic Linkage, 1994, John Hopkins University Press, 188-98), incorporated by reference herein, was used to estimate haplotype frequencies under $H_0$ (no linkage) and $H_1$ (allelic linkage allowed). It was found that the elaborate allele grouping strategy had some advantages over commonly used methods, in that disequilibrium was detected between almost all pairwise combinations of markers examined and there was good correlation between disequilibrium and physical distance.

More specifically, the E.H. program of Xie and Ott was used to determine maximum likelihood estimates of disequilibrium ($D_j$) between each pairwise combination of alleles, where $D_{ij} = h_{ij} - p_i q_j$ are the frequencies for allele i at locus 1 and allele j at locus 2 respectively, and $h_{ij}$ is the frequency of the haplotype ij. The program calculated maximum likelihood values for the haplotype frequencies (and hence allele frequencies) under $H_0$ (no association) and haplotype frequencies under $H_1$ (allelic association allowed).

For markers with greater than two alleles, the E. H. estimate for allele frequencies correlated poorly with the allele frequencies as estimated directly from the sample population, and therefore gave no confidence to the $D_{ij}$ estimates given. It was therefore necessary to group alleles of the multi-allelic markers into a biallelic system. Analysis of the markers in a biallelic format has the added advantages that the notation $\hat{D}_{ij}$, $p_j$, and $q_j$ can be simplified to D, p, and q respectively, where p and q are defined to be the frequencies of the rarer alleles at both loci (such that without loss of generality $p \leq q \leq 0.5$), and $\hat{D}$ is the estimated disequilibrium between those alleles.

Under a biallelic system, power is also much simpler to determine using equations as detailed by Hill (Hill, Heredity, 33: 229-39 (1974)). In addition, the sign of $\hat{D}$ becomes informative, such that $\hat{D} > 0$ when the rarer alleles at each of the two loci are associated, and D < 0 when the rare allele at one locus is associated with the common allele at the other locus.

Because the method of allele grouping clearly affected the power to detect disequilibrium (Zouros, et al., Genet. 85: 543-50 (1977); Weir, et al., Genet. 88: 633-42 (1976)), a preliminary analysis was conducted to ensure that the grouping did not mask disequilibrium between subsets of alleles. In this analysis, $\delta_{ij} = (O'_{ij} - E_{ij})/\sqrt{E_{ij}}$ was calculated for each haplotype, where $E_{ij}$ is the expected number of haplotypes ij assuming equilibrium ($E_{ij} = 2n\, p_i q_j$, where n = number of individuals in the study), and $O'_{ij}$ is a basic estimate for the observed haplotype count determined as follows. All genotypes that could be unambiguously resolved were haplotype counted. Each double heterozygote ($i_1 i_2/j_1 j_2$) could be resolved into two possible haplotype sets, $[i_1 j_1, i_2 j_2]$ or $[i_1 j_2, i_2 j_1]$. Using the haplotype frequencies as estimated from the unambiguous haplotype count, the probability of each set was calculated and used as a "partial" count. In this way the ambiguous genotypes were also haplotype counted, and the total counts (ambiguous plus unambiguous) constituted the $O'_{ij}$'s used in $\delta_{ij}$. Once established, the magnitude and sign of the $\delta_{ij}$'s were used to determine which allelic combinations showed greatest deviation from the null hypothesis of no association. This information was used to group alleles at the multiallelic loci into biallelic systems to enable efficient use of the E.H. program.

In order to compare the degree of disequilibrium between different pairwise combinations of loci, a frequency independent measure of disequilibrium ($\tilde{D}$, the proportion of maximum possible disequilibrium in the given direction) was calculated, where $\tilde{D} = D/|D_{max}|$ (Thompson, et al., Am. J. Hum. Genet. 42: 113-24 (1988)). The relationship between p and q are such that $p \leq q \leq 0.5$, and it can therefore be written that $-pq \leq D \leq p(1-q)$ such that when D < 0, $D_{max} = -pq$ and when $\hat{D} > 0$, $D_{max} = p(1-q)$. Output from the E.H. program included log-likelihoods for the maximum likelihood parameter values under $H_0$ and $H_1$, and since $-2\ln (L_0/L_1) \sim X^2_{1.}$ where $L_0$ and $L_1$ are the likelihoods under $H_0$ and $H_1$, p- values could then be determined for each test.

The asymptotic variance for $\hat{D}$, under $H_0$: D = 0 and $H_1$ were computed using the formula as defined by Hill (Heredity 33: 229-39 (1974)) for genotypic data. Using these, the power for each pairwise comparison could be calculated.

Common haplotypes containing all 8 loci were identified from the preliminary analysis of $\delta_{ij}$ described above, and backed up by the magnitude and sign of the disequilibria once the alleles at the multiallelic loci had been grouped. For these loci, the allele in the group which contributed most to the disequilibrium has been identified on the haplotype. To estimate the population haplotype frequencies, rates of carriage of at least one copy of the relevant alleles in the population were determined. These do not represent true haplotypes since phase is unknown. Monte Carlo simulation techniques were used to test for significant deviation from a simulated null distribution for these combined carriages under the assumption of no association.

## Example 3 <u>Estimation of Linkage Disequilibrium in the IL-1 Gene Cluster</u>

A number of biallelic and multiallelic markers in and around the IL-1 genes have been identified. However, the extent of linkage disequilibrium between the markers, and the prevalence of multimarker haplotypes in the general population have not until now been identified.

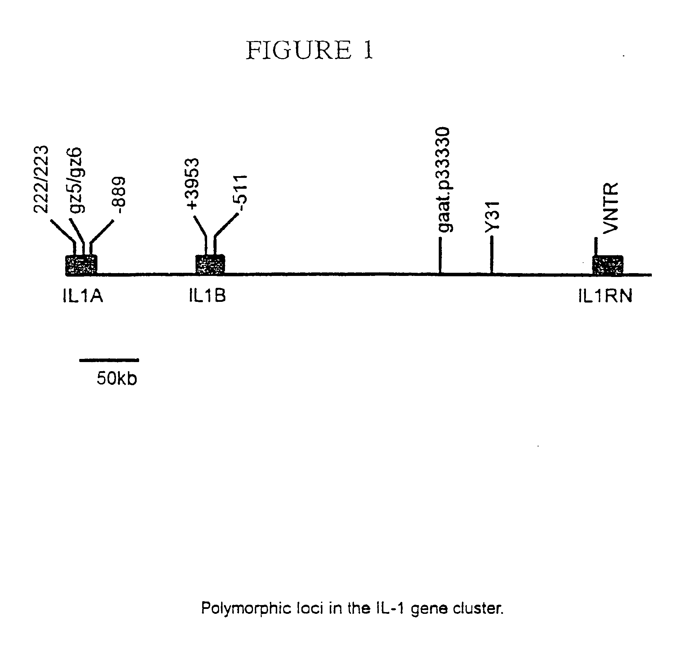

Figure 1 shows the relative positions of the 8 marker loci used in this study. DNA samples from 212 unrelated healthy volunteers were genotyped for each of these markers, and the resulting estimates of allele frequencies are shown in Table 4.

### Table 4. Estimated frequencies of marker alleles

| 222/223 | freq. | gz5/gz6 I | freq. | -889 | freq. | +3953 | freq. |
|---------|-------|-----------|-------|------|-------|-------|-------|
| 1 (126 bp) | 0.005 | 1 (79 bp) | 0.003 | 1 (Ncol) | 0.714 | 1 (2 Taql) | 0.812 |
| 2 (128 bp) | 0.018 | 2 (82 bp) | 0.005 | 2 | 0.286 | 2 | 0.188 |
| 3 (130 bp) | 0.378 | 3 (88 bp) | 0.676 | | | | |
| 4 (132 bp) | 0.299 | 4 (91 bp) | 0.316 | | | | |

## EP 0 983 385 B1

(continued)

| 222/223 | freq. | gz5/gz6 I | freq. | -889 | freq. | +3953 | freq. |
|---|---|---|---|---|---|---|---|
| 5 (134 bp) | 0.016 | | | | | | |
| 6 (136 bp) | 0.208 | | | | | | |
| 7 (138 bp) | 0.055 | | | | | | |
| 8 (140 bp) | 0.003 | | | | | | |
| 9 (142 bp) | 0.010 | | | | | | |
| 10 (144 bp) | 0.008 | | | | | | |
| *total | 384 | | 392 | | 398 | | 398 |
| -511 | freq. | gaat.p33330 | freq. | Y31 | freq. | VNTR | freq. |
| 1 | 0.618 | 1 (189 bp) | 0.658 | 1 (148 bp) | 0.092 | 1 | 0.744 |
| 2 (Bsu36l) | 0.382 | 2 (193 bp) | 0.002 | 2 (158 bp) | 0.008 | 2 | 0.256 |
| | | 3 (197 bp) | 0.255 | 3 (160 bp) | 0.454 | | |
| | | 4 (201 bp) | 0.084 | 4 (162 bp) | 0.062 | | |
| | | | | 5 (164 bp) | 0.003 | | |
| | | | | 6 (166 bp) | 0.122 | | |
| | | | | 7 (168 bp) | 0.035 | | |
| | | | | 8 (170 bp) | 0.030 | | |
| | | | | 9 (172 bp) | 0.095 | | |
| | | | | 10 (174 bp) | 0.087 | | |
| | | | | 11 (176 bp) | 0.003 | | |
| | | | | 12 (178 bp) | 0.011 | | |
| | 398 | | 404 | | 370 | | 398 |

*number of chromosomes analyzed
Note - Allele names (and sizes) are given in boldface.

To determine the linkage disequilibria between pairwise combinations of loci, the computer program of Xie and Ott was used. This program was found to be most efficient when used with biallelic systems, therefore alleles at the multiallelic loci were grouped in the most appropriate way for each pairwise comparison, such that disequilibrium between subsets of alleles was not masked.

In Table 5, the disequilibria between pairs of loci are expressed as D, the ratio of D to its maximum value $D_{max}$ and are shown together with the approximate physical distances between the loci in kilobase pairs.

**Table 5. Disequilibrium ($\tilde{D} = \hat{D}/|D_{max}|$) and physical distances between markers**

| | 222/223 | gz5/gz6 | -889 | +3953 |
|---|---|---|---|---|
| **222/223** | - | +0.872 | +0.829 | +0.710 |
| **gz5/gz6** | 2.5 | - | -0.889 | -0.695 |
| **-889** | 7 | 4.5 | - | +0.804 |
| **+3953** | 55 | 55 | 50 | - |
| **-511** | 60 | 60 | 55 | 4.5 |
| **gaat.p33330** | 260 | 260 | 255 | 205 |
| **Y31** | 310 | 310 | 305 | 255 |
| **VNTR** | 380 | 380 | 375 | 325 |

(continued)

|  | 222/223 | gz5/gz6 | -889 | +3953 |
|---|---|---|---|---|
|  | -511 | gaat.p33330 | Y31 | VNTR |
| 222/223 | +0.535 | +0.433 | +0.364 | -0.499 |
| gz5/gz6 | +0.540 | +0.517 | -0.503 | +0.286 |
| -889 | -0.264 | +0.337 | +0.318 | -0.207 |
| +3953 | -0.617 | +0.409 | -0.475 | -0.439 |
| -511 | - | +0.691 | -0.456 | +0.448 |
| gaat.p33330 | 200 | - | +0.639 | +0.442 |
| Y31 VNTR | 250 | 50 | - | -0.765 |
|  | 320 | 120 | 70 | - |
| Note - disequilibrium values are shown at the top right, approximate physical distances in Kb are shown at the bottom left. Intergenic distances are given to the nearest 5 Kb. |||||

Table 6 shows the power to detect 50% $D_{max}$ for each locus combination, and the p values for each corresponding D.

### Table 6. Power to detect 50% $D_{max}$ and p values of $-2Ln (L_0L_1)$

|  | 222/223 | gz5/gz6 | -889 | +3953 |
|---|---|---|---|---|
| 22/223 | - | -100 (+) | -100(+) | 98(+) |
| gz5/gz6 | $<1 \times 10^{-10}$ | - | 87(-) | 60(-) |
| -889 | $<1 \times 10^{-10}$ | $\sim 3 \times 10^{-8}$ | - | $\sim 100(+)$ |
| +3953 | $\sim 1 \times 10^{-7}$ | $* \sim 9 \times 10^{-3}$ | $<1 \times 10^{-10}$ | - |
| -511 | $\sim 9 \times 10^{-10}$ | $\sim 4 \times 10^{-10}$ | $* \sim 9.4 \times 10^{-2}$ | $* \sim 2.6 \times 10^{-2}$ |
| gaat.p33330 | $\sim 9 \times 10^{-8}$ | $\sim 2 \times 10^{-9}$ | $* \sim 1.7 \times 10^{-2}$ | $\sim 5 \times 10^{-4}$ |
| Y31 | $\sim 1 \times 10^{-4}$ | $\sim 4 \times 10^{-4}$ | $\sim 6 \times 10^{-4}$ | $\sim 1 \times 10^{-7}$ |
| VNTR | $\sim 1 \times 10^{-3}$ | $\sim 1 \times 10^{-3}$ | $* \sim 3 \times 10^{-1}$ | $* \sim 1.2 \times 10^{-1}$ |
|  | -511 | gaat.p33330 | Y31 | VNTR |
| 22/223 | $\sim 100(+)$ | $\sim 100(+)$ | $\sim 100(+)$ | 93(-) |
| gz5/gz6 | $\sim 100(+)$ | $\sim 100(+)$ | 98(-) | $\sim 100(+)$ |
| -889 | 96(-) | 89(+) | $\sim 100(+)$ | 78(-) |
| +3953 | 79(-) | 97(+) | $\sim 100(+)$ | 52(-) |
| -511 | - | $\sim 100(+)$ | $\sim 100(-)$ | $\sim 100(+)$ |
| gaat.p33330 | $<1 \times 10^{-10}$ | - | 49(+) | $\sim 100(+)$ |
| Y31 | $\sim 2 \times 10^{-4}$ | $* \sim 7 \times 10^{-3}$ | - | 89(-) |
| VNTR | $\sim 8 \times 10^{-6}$ | $\sim 1 \times 10^{-9}$ | $2 \times 10^{-7}$ | - |
| Note - Power is shown at the top right with the sign of disequilibrium in brackets; pointwise p-values are shown (uncorrected) at the bottom left. For an overall significance level of p = 0.05, pointwise significance level is 0.0018 for 28 comparisons. *Not significant at p = 0.0018 threshhold |||||

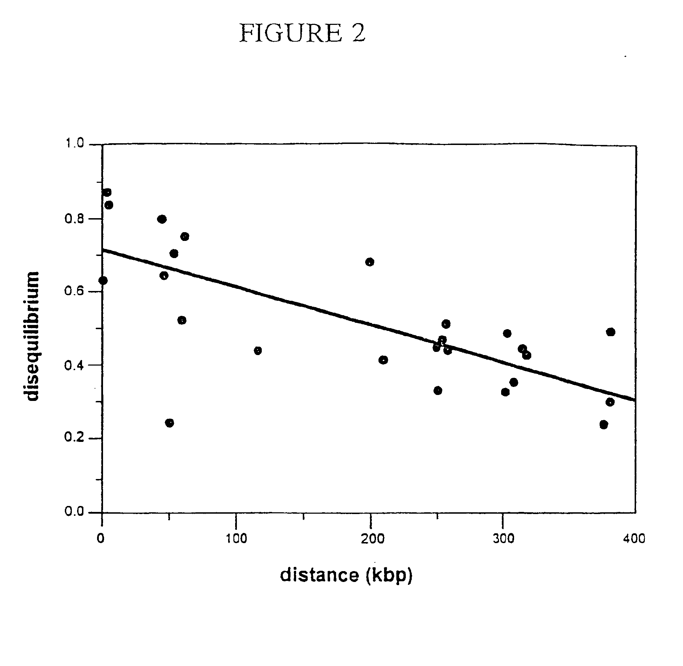

Significant linkage disequilibrium ($p_{corr} < 0.05$) was detected between most combinations of loci, with only a few exceptions. These include the comparisons between the VNTR and the more distant biallelic markers, +3953, and -889, in which the disequilibrium is in the negative direction and consequently the power is reduced (Table 6). The correlation between disequilibrium $\tilde{D}$ and physical distance was r = -0.752 (p < 0.0001, one tailed) (Figure 2).

In order to compare different grouping methods for the multiallelic markers, D was calculated for all the comparisons involving 222/223 using two additional grouping strategies. The first of these was a "common allele versus the rest" approach, and the second was a grouping based on allele size, using the bimodal distribution of allele frequency versus size which was observed for all the multiallelic markers examined. The results of this analysis are shown in Table 7, where $\tilde{D}$ values for the three grouping methods are compared.

**Table 7. $\tilde{D}$ values for three methods of grouping alleles at the multiallelic marker loci**

|  | $\delta_{11}$ | common vs. rest | allele size |
|---|---|---|---|
| **gz5/gz6** | 0.87 | 0.79 | 0.77 |
| **-899** | 0.83 | 0.81 | 0.98 |
| **+3953** | 0.71 | 0.74 | 0.77 |
| **-511** | 0.54 | *0.15 | 0.61 |
| **gaat.p33330** | 0.43 | *0.03 | 0.53 |
| **Y31** | 0.36 | * 0.12 | 0.16 |
| **VNTR** | 0.5 | 0.48 | * 0.04 |
| Note - Values are given for the disequilibrium between 222/223 and the other markers listed. <br> * indicates not significant at p = 0.05 level, even before correction for multiple testing. | | | |

It can be seen that the disequilibrium is not detected in several instances using these other grouping strategies, notably 222/223 with -511 and gaat.p33330 in the common versus rest approach, 222/223 with Y31 in both the common versus rest and allele size approaches, and 222/223 with VNTR in the allele size approach.

Examination of which alleles of the multiallelic loci were contributing greatest to the disequilibrium, from the determination of $\delta_{ij}$, revealed the existence of 2 haplotypes containing alleles of all 8 loci. These were confirmed by examination of the haplotype frequencies and disequilibrium values obtained after the grouping. The first haplotype: alleles 44112332 (expressed in chromosome order, see Fig. 1) is the most common (carriage of 34/198), and is present 7 times more frequently than expected (expected = 4.5/198) (p < 0.000001). The second haplotype: alleles 33221461 (carriage of 2/206) was present 4 times more frequently than expected (expected = 0.5/206), but this was not statistically significant (p ~ 0.106). However, examination of a larger sample size might assist increase the statistical significance of this finding.

The data presented indicate a significant degree of linkage disequilibrium across an approximately 400 Kb stretch of chromosome 2q13. The disequilibrium was strong both for the three markers within the IL-1$\alpha$ gene, as might be expected, but was also strong between some of the more distantly separated markers (-899/+3953; $\tilde{D}$ = +0.804, physical distance = 50 Kb) (Table 6). However, $\tilde{D}$ was considerably diminished between the extreme ends of the cluster. Within the IL-1 $\beta$ gene, a moderate value of $\tilde{D}$ (+3953/-511; $\tilde{D}$ = -0.617) was obtained, although this was not significant when corrected for multiple comparisons, probably reflecting the reduction in power when disequilibrium is in the negative direction (Thompson, et al., Am J. Hum. Genet. 42: 113-24 (1988)).

Overall, there is a good correlation between physical distance and linkage disequilibrium (Figure 2); r = -0.752. The reliability of r itself depends partly on the reliability of the estimates of both physical distance and D. Over the short distances, the physical distances are accurate since they are determined from known DNA sequence, whereas the longer range estimates are less precise. The power can be taken tentatively as one indicator of the reliability of $\tilde{D}$, since if power is low this indicates that the sample size was too small, and with low sample sizes the estimates for $\tilde{D}$ may be unreliable.

The success of the elaborate grouping strategy is indicated by Table 7, which shows several instances where disequilibrium between particular loci is apparently low or not detected when other commonly used grouping methods are employed. The disadvantages of the grouping strategy used here are that it is rather laborious since the information used for the grouping was based on an approximate estimate of the "observed" haplotype frequencies (see Example 2). For the more polymorphic markers the higher heterozygosity meant that the estimate of $\delta_{ij}$, was less precise since there was a higher proportion of ambiguous haplotypes. Notwithstanding this drawback, care was taken to take into account both the sign and magnitude of $\delta_{ij}$, and the frequencies of the alleles concerned.

The method could be simplified, in a sufficiently large study, by just considering the unambiguous haplotypes when determining the grouping. The determination of $\delta_{ij}$ uses the maximum amount of prior knowledge for the grouping of the multiallelic markers, and this may be the reason why disequilibrium between almost all pairwise combinations of markers was detected.

The two haplotypes containing all 8 markers, as well as other shorter haplotypes, are of particular interest since it is likely that particular combinations of alleles of the IL-1 genes may act in concert to determine an overall inflammatory phenotype. An understanding of which markers are in strong linkage disequilibrium not only allows for more rational design of genetic studies but also may provide clues to disease mechanism.

Additional alleles may also be part of the IL-1 (44112332) haplotype. We have previously shown that 5 novel markers were associated with allele 2 of the VNTR marker of the IL1RN gene (Clay, et al., Hum. Genet. 97: 723-6 (1996)). It is probable that these five alleles are in linkage disequilibrium, although confirmation by statistical analyses has not yet been performed. Therefore, the IL-1 (44112332) haplotype may also include these five markers.

Similarly, Guasch found 3 additional alleles in IL1RN (Guasch, et al, Cytokine 8: 598-602 (1997)) which appeared at the same frequencies in a small sample (12 individuals). One of the Guasch alleles (the MspI allele) is the 8006 allele of the Clay study. Therefore, the additional two alleles by Guasch (MwoI and SspI alleles) may be part of the IL-1 (4112332) haplotype discovered herein. However, this should be confirmed as described herein because the very small sample size may have produced misleading results.

Guasch also worked with three alleles in the IL1B gene. The alleles were allele 5887, which probably corresponds to the Taq allele at position +3953 of IL1B described herein, as well as a 5810 (BsoFI) allele and a 1903 (AluI) allele. These three alleles are said to be in "poor linkage disequilibrium" but accurate typing with a larger sample size may indicate that the AluI allele is also part of the IL-1 (4412332) haplotype since the allele frequencies of the TaqI and AluI alleles are similar (0.78/0.22 vs. 0.74/0.26). However, the linkage disequilibrium appears weaker than the IL1RN alleles described by Guasch.

Therefore, in addition to the alleles identified herein, the IL-1 (44112332) haplotype may contain the following alleles:

| | |
|---|---|
| allele 2 of the 1731 marker of the IL1RN gene | (A at position 1731) |
| allele 2 of the 1812 marker of the IL1RN gene | (A at position 1812) |
| allele 2 of the 1868 marker of the IL1RN gene | (G at position 1868) |
| allele 2 of the 1887 marker of the IL1RN gene | (C at position 1887) |
| allele 2 of the 8006 marker of the IL1RN gene | (contains a HpaII or MspI site) |
| allele 2 of the 8061 marker of the IL1RN gene | (lacks a MwoI site) |
| allele 2 of the 9589 marker of the IL1RN gene | (contains an SspI site) |

Furthermore, the following PCR primers may be used to amplify these alleles:
TTACGCAGATAAGAACCAGTTTGG (SEQ ID NO. 17)
TTTCCTGGACGCTTGCTCACCAG (SEQ ID NO. 18)
(used for 1731, 1812, 1868, and 1887)
TTCTATCTGAGGAACAACCAACTAGTAGC (SEQ ID NO. 19)
CACCAGACTTGACACAGGACAGGCACATC (SEQ ID NO. 20)
(used for 8006)
CGACCCTCTGGGAGAAAATCCAGCAAG (SEQ ID NO. 21)
(used with SEQ ID NO. 20 for 8006)
ACACAGGAAGGTGCCAAGCA (SEQ ID NO. 22)
TGCAGACAGACGGGCAAAGT (SEQ ID NO. 23)
(used for 8006 and 9589)
TTGTGGGGACCAGGGGAGAT (SEQ ID NO. 24), and
AGCCTGGCACTCTGCTGAAT (SEQ ID NO. 25)
(used for 9589).

## Example 4 The IL-1 (44112332) Haplotype is Associated with Diabetic Nephropathy

The presence of the two haplotypes described herein was investigated in healthy and diseased populations to determine if the haplotypes were associated with inflammatory disease. 81 non-insulin dependant diabetes mellitus (NIDDM) patients with nephropathy were compared with 198 ethnically matched healthy subjects in example 3 and 147 NIDDM patients without nephropathy. Genotyping was carried out as in example 1.

The IL-1 (44112332) haplotype was carried by 24 of 79 of the NIDDM nephropathy patients and 25 of 141 NIDDM without nephropathy patients. However, the second haplotype (33221461) was not found in the nephropathy patients (0/81). The IL-1 (44112332) haplotype was significantly over represented in the patient group compared with the healthy control group (24/79 vs. 34/198; p = 0.015) and the NIDDM without nephropathy group (24/79 vs. 25/141; p = 0.03). Therefore, the haplotype is associated with inflammatory disease.

**Example 5 An IL-1 Haplotype is Associated with Inflammatory Disease**

This is a prophetic example. Other diseases are examined as per Example 4. The IL-1 (44112332) haplotype is found to be associated with coronary artery disease, osteoporosis, nephropathy in diabetes mellitus, alopecia areata, Graves disease, systemic lupus erythematosus, lichen sclerosis and ulcerative colitis.
Likewise, the IL-1 (33221461) haplotype is associated with periodontal disease, juvenile chronic arthritis, psoriasis, insulin dependant diabetes and diabetic retinopathy.

**Example 6 Novel Markers are Linked to an IL-1 Haplotype**

This is a prophetic example. Additional markers are identified by sequence and restriction enzyme analysis of the 2q13-14 region. These new markers are identified as belonging to an IL-1 haplotype in the manner described in Examples 2 and 3.

**Example 7 The IL-1 (41112332) Haplotype Is Used to Predict Disease Susceptibility**

This is a prophetic example. A patient with a family history of ulcerative colitis is genotyped for the presence of the IL-1 (44112332) haplotype. Genotyping is performed as in Example 1 and the patient is determined to carry one or more alleles of the haplotype. The patient is thus treated with IL-1 antagonists to prevent disease.
A second patient with a family history of coronary artery disease is genotyped at the IL-1 gene cluster. The patient is found to carry one or more alleles of the IL-1 (44112332) haplotype and be homozygous for the VNTR allele 2. Thus, the patient is 5.4 times as likely to develop coronary artery disease as the general population and is treated vigorously to prevent disease.

**Example 8 Additional Haplotypes are Statistically Significant**

This is a prophetic example. An additional 400 chromosomes are typed as per Example 1 and linkage disequilibrium assessed as per Example 2. The IL-1 (33221461) haplotype is found to be present about 4 times more frequently than expected (p ~ 0.05).
In a similar manner, the following markers are determined to be present in the IL-1 (44112332) haplotype (p << 0.05).
allele 2 of the 1731 marker of the IL1RN gene
allele 2 of the 1812 marker of the IL1RN gene
allele 2 of the 1868 marker of the IL1RN gene
allele 2 of the 1887 marker of the IL1RN gene
allele 2 of the 8006 marker of the IL1RN gene
allele 2 of the 8061 marker of the IL1RN gene
allele 2 of the 9589 marker of the IL1RN gene
All documents cited herein are incorporated by reference and are separately listed here for convenience:
United States Patent No. 4,582,788
United States Patent No. 4,666,828
United States Patent No. 4,801,531
United States Patent No. 5,110,920
United States Patent No. 5,268,267
U.S. Application No. 08/813,416
U.S. Application No. 08/628,282
GB Application No. 9618960.0
Blakemore, et al., Hum. Genet. 97(3): 369-74 (1996)
Blakemore, et al., J. Clin. Endocrinol. 80(1): 111-5 (1995)
Blakemore, et al., Arthritis Rheum. 37: 1380-85 (1994)
Clay, et al., Hum. Genet. 97: 723-6 (1996)
Clay, et al., Hum. Genet. 94: 407-10 (1994)

di Giovine, et al., Hum. Molec. Genet. 1(6): 450 (1992)

di Giovine, et al., Cytokine 7(6): 606 (1995)

Guasch, et al, Cytokine 8: 598-602 (1997)

Handbook of Human Genetic Linkage, 1994, John Hopkins University Press

Hill, Heredity, 33: 229-39 (1974)

Pociot, et al., Eur. J. Clin. Invest. 22: 396-402 (1992)

Mansfield, et al., Gastoenterol. 106(3): 637-42 (1994)

McDowell, et al., Br. J Rheumatol. 32(Supp 1): 182 (1983)

McDowell, et al., Arthritis Rheum. 38: 221-28 (1995)

Moling et al., Scand. J. Immunol. 27:705-16 (1988)

Nicklin, et al., Genomics 19: 382-4 (1994)

Nothwang et al. Genomics (in press)

Pociot, et al., Eur J. Clin. Invest. 22: 396-402 (1992)

Spurr, et al., Cytogenet. & Cell Genet. (in press, 1996)

Tarlow, et al., Hum. Genet. 91: 403-4 (1993)

Tarlow, et al., J. Invest. Dermatol. 103: 387-90 (1994)

Thompson, et al., Am. J. Hum. Genet. 42: 113-24 (1988)

Todd & Naylor, Nucleic Acids Res. 19: 3756 (1991)

Weir, et al., Genet. 88: 633-42 (1976)

Wilson, et al., Hum. Molec. Genet. 1(5): 353 (1992)

Xie and Ott, HANDBOOK OF HUMAN GENETIC LINKAGE, John Hopkins Un. Press, pp. 188-98 (1994) Zouros, et al., Genet. 85: 543-50 (1977)

Zuliani, et al., Am. J Hum. Genet. 46: 963-69 (1990)

**Claims**

1.  A method for determining a patient's susceptibility to an inflammatory disorder, said method comprising:

    detecting the presence of an IL-1 (44112332) haplotype in a biological sample obtained from a patient,

    wherein the IL-1 (44112332) haplotype is defined by a plurality of alleles selected from the group consisting of:

    allele 4 of a 222/223 marker of IL1A;
    allele 4 of a gz5/gz6 marker of IL1A;
    allele 1 of a -889 marker of IL1A;
    allele 1 of a +3953 marker of IL1B;
    allele 2 of a -511 marker of IL1B;
    allele 3 of a gaat.p33330 marker;
    allele 3 of a Y31 marker; and
    allele 2 of a VNTR marker of IL1RN;

    and wherein the presence of said IL-1 (44112332) haplotype indicates said patient's susceptibility to an inflammatory disorder, wherein said inflammatory disorder is selected from the group consisting of coronary artery disease, osteoporosis, nephropathy in diabetes mellitus, alopecia areata, Graves disease, systemic lupus erythamatosus, lichen sclerosis, and ulcerative colitis.

2.  The method of claim 1, wherein said detecting is done by amplifying said biological sample with PCR using at least one primer selected from the group consisting of:
    ATGTATAGAATTCCATTCCTG (SEQ ID NO. 1),
    TAAAATCAAGTGTTGATGTAG (SEQ ID NO. 2),
    GGGATTACAGGCGTGAGCCACCGCG (SEQ ID NO.3),
    TTAGTATTGCTGGTAGTATTCATAT (SEQ ID NO.4),
    TGTTCTACCACCTGAACTAGG (SEQ ID NO. 5),
    TTACATATGAGCCTTCCATG (SEQ ID NO. 6),
    CTCAGGTGTCCTCGAAGAAATCAAA (SEQ ID NO.7),
    GCTTTTTTGCTGTGAGTCCCG (SEQ ID NO.8),
    TGGCATTGATCTGGTTCATC (SEQ ID NO.9),

GTTTAGGAATCTTCCCACTT (SEQ ID NO. 10),
GAGGCGTGAGAATCTCAAGA (SEQ ID NO.11),
GTGTCCTCAAGTGGATCTGG (SEQ ID NO. 12),
GGGCAACAGAGCAATGTTTCT (SEQ ID NO.1),
CAGTGTGTCAGTGTACTGTT (SEQ ID NO. 14),
CTCAGCAACACTCCTAT (SEQ ID NO. 15), and
TCCTGGTCTGCAGGTAA (SEQ ID NO. 16).

3. A diagnostic kit for the prediction of inflammatory disorder, said kit comprising at least two oligonucleotides selected from the group consisting of.
ATGTATAGAATTCCATTCCTG (SEQ ID NO.1),
TAAAATCAAGTGTTGATGTAG (SEQ ID NO. 2),
GGGATTACAGGCGTGAGCCACCGCG (SEQ ID NO.3),
TTAGTATTGCTGGTAGTATTCATAT (SEQ ID NO.4),
TGTTCTACCACCTGAACTAGG (SEQ ID NO. 5),
TTACATATGAGCCTTCCATG (SEQ ID NO. 6),
CTCAGGTGTCCTCGAAGAAATCAAA (SEQ ID NO.7),
GCTTTTTTGCTGTGAGTCCCG (SEQ ID NO.8),
TGGCATTGATCTGGTTCATC (SEQ ID NO.9),
GTTTAGGAATCTTCCCACTT (SEQ ID NO. 10),
GAGGCGTGAGAATCTCAAGA (SEQ ID NO.11),
GTGTCCTCAAGTGGATCTGG (SEQ ID NO. 12),
GGGCAACAGAGCAATGTTTCT (SEQ ID NO.13),
CAGTGTGTCAGTGTACTGTT (SEQ ID NO. 14),
CTCAGCAACACTCCTAT (SEQ ID NO. 15),
TCCTGGTCTGCAGGTAA (SEQ ID NO. 16), and
TTACGCAGATAAGAACCAGTTTGG (SEQ ID NO.17).

4. The diagnostic kit of claim 3, further comprising a control sample.

5. The diagnostic kit of claim 4, wherein said control sample comprises one or more alleles selected from the group consisting of
alleles of a 222/223 marker of IL 1A;
alleles of a gz5/gz6 marker of IL 1A;
alleles of a -889 marker of IL1A;
alleles of a +3953 marker of IL1B;
alleles of a -511 marker of IL1B;
alleles of a gaat.p33330 marker;
alleles of a Y31 marker; and
alleles of a VNTR marker of IL1RN.

6. The diagnostic kit of claim 3, wherein said oligonucleotide comprises a detectable label.

7. The diagnostic kit of claim 6, further comprising DNA sampling reagents, and PCR amplification reagents.

8. The diagnostic kit of claim 7, further comprising DNA sampling means.

**Patentansprüche**

1. Verfahren zur Bestimmung der Anfälligkeit eines Patienten für eine entzündliche Erkrankung, wobei das Verfahren aufweist:

Nachweisen des Vorliegens eines IL-1 (44112332)-Haplotyps in einer von einem Patienten erhaltenen biologischen Probe,

wobei der IL-1 (44112332)-Haplotyp durch eine Mehrzahl von Allelen definiert wird, die ausgewählt werden aus der Gruppe, die besteht aus:

Allel 4 eines 222/223-Markers von IL1A;
Allel 4 eines gz5/gz6-Markers von IL1A;
Allel 1 eines -889-Markers von IL1A;
Allel1 eines +3953-Markers von IL1 B;
Allel 2 eines -511-Markers von IL1 B;
Allel 3 eines gaat.p33330-Markers;
Allel 3 eines Y31-Markers; und
Allel 2 eines VNTR-Markers von IL1 RN;

und wobei die Anwesenheit des IL1 (44112332)-Haplotyps auf die Anfälligkeit des Patienten für eine entzündliche Erkrankung hinweist, wobei die entzündliche Erkrankung ausgewählt ist aus der Gruppe, die aus koronarer Herzkrankheit, Osteoporose, Nierenerkrankung bei Diabetes mellitus, Alopecia areata, Basedeow'-Krankheit, systemischem Lupus erythematosus, Lichensklerose und Colitis ulcerosa besteht.

2. Verfahren nach Anspruch 1, wobei das Nachweisen durch Amplifizieren der biologischen Probe mit PCR durchgeführt wird, wobei mindestens ein Primer verwendet wird, der ausgewählt wird aus der Gruppe, die besteht aus:

ATGTATAGAATTCCATTCCTG (SEQ ID NO.1),
TAAAATCAAGTGTTGATGTAG (SEQ ID NO. 2),
GGGATTACAGGCGTGAGCCACCGCG (SEQ ID NO.3),
TTAGTATTGCTGGTAGTATTCATAT (SEQ ID NO.4),
TGTTCTACCACCTGAACTAGG (SEQ ID NO. 5),
TTACATATGAGCCTTCCATG (SEQ ID NO. 6),
CTCAGGTGTCCTCGAAGAAATCAAA (SEQ ID NO.7),
GCTTTTTTGCTGTGAGTCCCG (SEQ ID NO.8),
TGGCATTGATCTGGTTCATC (SEQ ID NO.9),
GTTTAGGAATCTTCCCACTT (SEQ ID NO. 10),
GAGGCGTGAGAATCTCAAGA (SEQ ID NO.11),
GTGTCCTCAAGTGGATCTGG (SEQ ID NO. 12),
GGGCAACAGAGCAATGTTTCT (SEQ ID NO.13),
CAGTGTGTCAGTGTACTGTT (SEQ ID NO. 14),
CTCAGCAACACTCCTAT (SEQ ID NO. 15), und
TCCTGGTCTGCAGGTAA (SEQ ID NO. 16).

3. Diagnostischer Kit für die Voraussage einer entzündlichen Erkrankung, wobei der Kit mindestens zwei Oligonucleotide aufweist, die ausgewählt sind aus der Gruppe, die besteht aus:

ATGTATAGAATTCCATTCCTG (SEQ ID NO. 1),
TAAAATCAAGTGTTGATGTAG (SEQ ID NO. 2),
GGGATTACAGGCGTGAGCCACCGCG (SEQ ID NO.3),
TTAGTATTGCTGGTAGTATTCATAT (SEQ ID NO.4),
TGTTCTACCACCTGAACTAGG (SEQ ID NO. 5),
TTACATATGAGCCTTCCATG (SEQ ID NO. 6),
CTCAGGTGTCCTCGAAGAAATCAAA (SEQ ID NO.7),
GCTTTTTTGCTGTGAGTCCCG (SEQ ID NO.8),
TGGCATTGATCTGGTTCATC (SEQ ID NO.9),
GTTTAGGAATCTTCCCACTT (SEQ ID NO. 10),
GAGGCGTGAGAATCTCAAGA (SEQ ID NO.11),
GTGTCCTCAAGTGGATCTGG (SEQ ID NO. 12),
GGGCAACAGAGCAATGTTTCT (SEQ ID NO.13),
CAGTGTGTCAGTGTACTGTT (SEQ ID NO. 14),
CTCAGCAACACTCCTAT (SEQ ID NO. 15),
TCCTGGTCTGCAGGTAA (SEQ ID NO.16), und
TTACGCAGATAAGAACCAGTTTGG (SEQ ID NO.17).

4. Diagnostischer Kit nach Anspruch 3, außerdem eine Kontrollprobe aufweisend.

5. Diagnostischer Kit nach Anspruch 4, wobei die Kontrollprobe ein oder mehrere Allele aufweist, die ausgewählt sind aus der Gruppe, die besteht aus

Allelen eines 222/223-Markers von IL1A;
Allelen eines gz5/gz6-Markers von IL1A;
Allelen eines -889-Markers von IL1A;
Allelen eines +3953-Markers von IL1B;
Allelen eines -511-Markers von IL1B;
Allelen eines gaat.p33330-Markers;
Allelen eines Y31-Markers; und
Allelen eines VNTR-Markers von IL1RN.

6. Diagnostischer Kit nach Anspruch 3, wobei das Oligonucleotid eine nachweisbare Markierung aufweist.

7. Diagnostischer Kit nach Anspruch 6, außerdem DNA-Probenahmereagenzien und PCR-Amplifikationsreagenzien aufweisend.

8. Diagnostischer Kit nach Anspruch 7, außerdem eine DNA-Probenahmeeinrichtung aufweisend.

**Revendications**

1. Procédé de détermination d'une susceptibilité d'un patient à un trouble inflammatoire, ledit procédé comprenant :

la détection de la présence d'un haplotype (44112332) de l'IL-1 dans un échantillon biologique obtenu d'un patient,
dans lequel l'haplotype (44112332) de l'IL-1 est défini par une pluralité d'allèles choisis dans le groupe constitué par :

l'allèle 4 d'un marqueur 222/223 de l'IL1A ;
l'allèle 4 d'un marqueur gz5/gz6 de l'IL1A ;
l'allèle 1 d'un marqueur -889 de l'IL1A ;
l'allèle 1 d'un marqueur +3953 de l'IL1B ;
l'allèle 2 d'un marqueur -511 de l'IL1B ;
l'allèle 3 d'un marqueur gaat.p33330 ;
l'allèle 3 d'un marqueur Y31 ; et
l'allèle 2 d'un marqueur VNTR de l'IL1RN ;

et dans lequel la présence dudit haplotype (44112332) de l'IL-1 indique la susceptibilité dudit patient à un trouble inflammatoire, ledit trouble inflammatoire étant choisi dans le groupe constitué par une maladie coronarienne, l'ostéoporose, une néphropathie dans le diabète sucré, la pelade, la maladie de Graves, le lupus érythémateux systémique, le sclérolichen, et la rectocolite ulcéro-hémorragique.

2. Procédé selon la revendication 1, dans lequel ladite détection est effectuée en amplifiant ledit échantillon biologique avec une PCR utilisant au moins une amorce choisie dans le groupe constitué par :

ATGTATAGAATTCCATTCCTG (SET ID NO.1),
TAAAATCAAGTGTTGATGTAG (SEQ ID NO.2),
GGGATTACAGGCGTGAGCCACCGCG (SEQ ID NO.3),
TTAGTATTGCTGGCTAGTATTCATAT (SEQ ID NO.4).
TGTTCTACCACCTGAACTAGG (SEQ ID NO. 5),
TTACATATGAGCCTTCCATG (SEQ ID NO.6),
CTCAGGTGTCCTCGAAGAAATCAAA (SEQ ID NO.7),
GCTTTTTTGCTGTGAGTCCCG (SEQ ID NO.8),
TGGCATTGATCTGGTTCATC (SEQ ID NO.9),
GTTTAGGAATCTTCCCACTT (SEQ ID NO.10),
GAGGCGTGAGAATCTCAAGA (SEQ ID NO.11),
GTGTCCTCAAGTGGATCTGG (SEQ ID NO. 12),
GGGCAACAGAGCAATGTTTCT (SEQ ID NO.13),

CAGTGTGTCAGTGTACTGTT (SEQ ID NO.14),
CTCAGCAAGACTCCTAT (SEQ ID NO.15), et
TCCTGGTCTGCAGGTAA (SEQ ID NO. 16).

3. Kit de diagnostic pour la prédiction d'un trouble inflammatoire, ledit kit comprenant au moins deux oligonucléotides choisis dans le groupe constitué par :

ATGTATAGAATTCCATTCCTG (SEQ ID NO.1),
TAAAATCAAGTGTTGATGTAG (SEQ ID NO.2),
GGGATTACAGGCGTGAGCCACCGCG (SEQ ID NO.3),
TTAGTATTGCTGGTAGTATTCATAT (SEQ ID NO.4).
TGTTACCACCTGAACTAGG (SEQ ID NO.5),
TTACATATGAGCCTTCCATG (SEQ ID NO. 6),
CTCAGGTGTCCTCGAAGAAATCAAA (SEQ ID NO.7),
GCTTTTTTGCTGTGAGTCCCG (SEQ ID NO.8),
TGGCATTGATCTGGTTCATC (SEQ ID NO.9).
GTTTAGGAATCTTCCCACTT (SEQ ID NO.10),
GAGGCGTGAGAATCTCAAGA. (SEQ ID NO.11),
GTGTCCTCAAGTGGATCTGG (SEQ ID NO.12).
GGGCAACAGAGCAATGTTTCT (SEQ ID NO.13),
CAGTGTGTCAGTGTACTGTT (SEQ ID NO. 14),
CTCAGCAACACTCCTAT (SEQ ID NO.15),
TCCTGGTCTGCAGGTAA (SEQ ID NO. 16), et
TTACGCAGATAAGAACCAGTTTGG (SEQ ID NO.17),

4. Kit de diagnostic selon la revendication 3, comprenant en outre un échantillon témoin.

5. Kit de diagnostic selon la revendication 4, dans lequel ledit échantillon témoin comprend un ou plusieurs allèles choisis dans le groupe constitués par :

les allèles d'un marqueur 222/223 de l'IL1A ;
les allèles d'un marqueur gz5/gz6 de l'IL1A ;
les allèles d'un marqueur -889 de l'IL1A ;
les allèles d'un marqueur +3953 de l'IL1B ;
les allèles d'un marqueur -511 de l'IL1B ;
les allèles d'un marqueur gaat.p33330 ;
les allèles d'un marqueur Y31 ; et
les allèles d'un marqueur VNTR de l'IL1RN.

6. Kit de diagnostic selon la revendication 3, dans lequel ledit oligonucléotide comprend un marqueur détectable.

7. Kit de diagnostic selon la revendication 6, comprenant en outre des réactifs d'échantillonnage d'ADN, et des réactifs d'amplification PCR.

8. Kit de diagnostic selon la revendication 7, comprenant en outre des moyens d'échantillonnage d'ADN.

# FIGURE 1

Polymorphic loci in the IL-1 gene cluster.

## FIGURE 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4582788 A
- US 5110920 A
- US 4801531 A
- US 4666828 A
- US 5268267 A
- US 813416 A
- US 08628282 A
- WO 9815653 A
- US 5686246 A
- US 628282 A
- GB 9618960 A

**Non-patent literature cited in the description**

- **Nicklin et al.** *Genomics,* 1994, vol. 19, 382-4
- **Molvig et al.** *Scand. J. Immunol.,* 1988, vol. 27, 705-16
- **Pociot et al.** *Eur. J. Clin. Invest.,* 1992, vol. 22, 396-402
- **Blakemore et al.** *Hum. Genet.,* 1996, vol. 97 (3), 369-74
- **Cork et al.** *J. Invest. Dermatol.,* 1995, vol. 104 (5), 15S-16S
- **Blakemore et al.** *J. Clin. Endocrinol.,* 1995, vol. 80 (1), 111-5
- **Blakemore et al.** *Arthritis Rheum.,* 1994, vol. 37, 1380-85
- **Clay et al.** *Hum. Genet.,* 1994, vol. 94, 407-10
- **Mansfield et al.** *Gastoenterol.,* 1994, vol. 106 (3), 637-42
- **McDowell.** *Arthritis Rheum.,* 1995, vol. 38, 221-28
- **di Giovine et al.** *Cytokine,* 1995, vol. 7, 606
- **Pociot et al.** *Eur J. Clin. Invest.,* 1992, vol. 22, 396-402
- **Tarlow JW et al.** *J. of Invest. Dermatol.,* 1994, vol. 103, 387-389
- **McDowell et al.** *Br. J. Rheumatol.,* 1983, vol. 32 (1), 182
- **Wilson et al.** *Hum. Molec. Genet.,* 1992, vol. 1 (5), 353
- **di Giovine et al.** *Hum. Molec. Genet.,* 1992, vol. 1 (6), 450
- **Clay et al.** *Human Genetics,* 1996, vol. 97 (6), 723-26
- **Todd ; Naylor.** *Nucleic Acids Res.,* 1991, vol. 19, 3756
- **Zuliani et al.** *Am. J. Hum. Genet.,* 1990, vol. 46, 963-69
- **McDowell et al.** *Arth. & Rheum.,* 1995, vol. 38, 221-8
- **di Giovine et al.** *Cytokine,* 1995, vol. 7 (6), 606
- **di Giovine.** *Hum. Molec. Genet.,* 1992, vol. 1 (6), 450
- **Spurr et al.** *Cytogenet.,* 1996, vol. 73, 255-73
- **Tarlow et al.** *Hum. Genet.,* 1993, vol. 91, 403-4
- **Nothwang et al.** *Genomics*
- Handbook of Human Genetic Linkage. John Hopkins University Press, 1994, 188-98
- **Hill.** *Heredity,* 1974, vol. 33, 229-39
- **Zouros et al.** *Genet.,* 1977, vol. 85, 543-50
- **Weir et al.** *Genet.,* 1976, vol. 88, 633-42
- **Thompson et al.** *Am. J. Hum. Genet.,* 1988, vol. 42, 113-24
- **Thompson et al.** *Am J. Hum. Genet.,* 1988, vol. 42, 113-24
- **Clay et al.** *Hum. Genet.,* 1996, vol. 97, 723-6
- **Guasch et al.** *Cytokine,* 1997, vol. 8, 598-602
- Handbook of Human Genetic Linkage. John Hopkins University Press, 1994
- **McDowell et al.** *Br. J Rheumatol.,* 1983, vol. 32 (1), 182
- **McDowell et al.** *Arthritis Rheum.,* 1995, vol. 38, 221-28
- **Moling et al.** *Scand. J. Immunol.,* 1988, vol. 27, 705-16
- **Spurr et al.** *Cytogenet. & Cell Genet.,* 1996
- **Tarlow et al.** *J. Invest. Dermatol.,* 1994, vol. 103, 387-90
- **Xie ; Ott.** HANDBOOK OF HUMAN GENETIC LINKAGE. John Hopkins Un. Press, 1994, 188-98
- **Zuliani et al.** *Am. J Hum. Genet.,* 1990, vol. 46, 963-69